# EUROPEAN PATENT SPECIFICATION

(11) **EP 2 777 690 B1**
(45) Date of publication and mention of the grant of the patent: **21.06.2017**
(21) Application number: 12847763.5
(22) Date of filing: 08.11.2012
(51) Int. Cl.: A61K 8/58, A61K 8/06, A61K 8/34, A61K 8/37, A61K 8/86, A61Q 17/04

(54) **OIL-IN-WATER TYPE EMULSIFIED SUNSCREEN COSMETIC**
ÖL-IN-WASSER-EMULGIERTES SONNENSCHUTZ-KOSMETIKUM
PRODUIT COSMÉTIQUE ÉCRAN SOLAIRE ÉMULSIFIÉ DE TYPE HUILE-DANS-EAU

(30) Priority: 11.11.2011 JP 2011247012; 05.11.2012 JP 2012243688
(43) Date of publication of application: 17.09.2014
(73) Proprietor: Shiseido Company, Ltd., Tokyo 104-0061 (JP)
(72) Inventor: NAOI, Kayoko, Yokohama-shi Kanagawa 224-8558 (JP)
(74) Representative: Henkel, Breuer & Partner
(86) International application number: PCT/JP2012/078952
(87) International publication number: WO 2013/069723

(56) References cited:
- EP-A1- 2 174 985
- EP-A1- 2 251 403
- EP-A2- 2 181 697
- WO-A1-2011/114773
- WO-A1-2012/070309
- JP-A- 2008 266 285
- JP-A- 2010 143 844
- HIDEFUMI ARAKI: 'Silicone Shushoku Shibosan Sekken (SMFS) ni yoru Atarashii a Gel-kei to sono Skin Care eno Oyo' FRAGRANCE JOURNAL vol. 37, no. 4, 15 April 2009, pages 6 - 7, XP008173338

## Description

### Technical Field

The present invention relates to an oil-in-water type emulsified sunscreen cosmetic. More particularly, the present invention relates to an oil-in-water type emulsified sunscreen cosmetic that, in spite of containing a large amount of an ultraviolet absorbent, has good spreadability on the skin and good affinity to the skin, has no stickiness after application on the skin, and additionally has excellent stability over time at low and high temperatures.

### Background Art

Protection of the skin against harmful ultraviolet rays is an important issue in skin care and body care, and to minimize adverse effects caused by ultraviolet rays on the skin, various UV care cosmetics have been developed. A sunscreen cosmetic, one type of UV care cosmetics, to which an ultraviolet absorber, an ultraviolet scattering agent and the like are added inhibits UVA and UVB from reaching the skin to thereby protect the skin against harmful ultraviolet rays (Non-patent document 1). Defense against ultraviolet is considered important these days, not only in outdoor activities such as bathing in summer and skiing in winter, but also in daily life, and so an ultraviolet protection effect is required even in common skin care cosmetics. Accordingly, in addition to the defense against ultraviolet, improvement in the feeling of use is required.

A sunscreen cosmetic which contains an ultraviolet absorber and an ultraviolet scattering agent in a gel structure substrate composed of a system of a specific organosiloxane derivative/a higher alcohol/water has been proposed (Patent document 1). The cosmetics of this type are said to be able to impart a refreshing and excellent feeling of use by a collapse of the gel structure on application on the skin.

However, in such a cosmetic having a gel structure composed of a system of an organosiloxane derivative/a higher alcohol/water, when the amount of the ultraviolet absorber to be added was increased in order to enhance a defense effect against ultraviolet, the cosmetic became less easy to spread or took more time to develop an affinity on application on the skin because most of ultraviolet absorbers were oil-soluble. Thus, the feeling of use tended to be impaired, for example stickiness was caused after application. Furthermore, when a large amount of an ultraviolet absorber was added, stability over time at low and high temperatures was decreased, the viscosity changed during storage, or creaming (condensation of an emulsion) was caused in some cases.

Accordingly, an oil-in-water type emulsified sunscreen cosmetic, which is excellent in stability over time and in a feeling of use even if it contains a large amount of an ultraviolet absorber for exhibiting a high sunscreen effect, is still desired.

Patent Document 2 discloses an oil-in-water emulsified sunscreen cosmetic providing good UV protection, and exhibiting good stability and sensation during use. The sunscreen cosmetic comprises (a) 1 to 6% by weight of a combination of POE stearic ester having a POE mole number of 20-120, sorbitan tristearate and glyceryl stearate having a HLB of 5-8, (b) 0.01 to 5% by weight of bis-ethylhexyloxyphenolmethoxyphenyltriazine and/or tert-butylmethoxybenzoylmethane as an oil-soluble UV absorber which is solid at room temperature, (c) 1 to 14% by weight of ethylhexyl methoxycinnamate and/or octocrylene as an oil-soluble UV absorber which is liquid at room temperature, (d) 0.1 to 5% by weight of phenylbenzimidazolesulfonic acid as a water-soluble UV absorber, and (e) a higher alcohol having 14-24 carbon atoms.

Patent Document 3 discloses a gel composition having good foamability and good cleansing effect, the gel composition comprising (a) an organosiloxane derivative, (b) a higher alcohol having 10-30 carbon atoms, and (c) water. The organosiloxane derivative is represented by the general formula R⁵-(CH₂)₂-[Si(R¹)(R²)-O]ₚ-Si(R³)(R⁴)-(CH₂)₂-A-COOM, in which R¹, R², R³ and R⁴ may be same or different and in each case represent a hydrogen atom, a C₁₋₆ alkyl group or a phenyl group; M is a hydrogen atom, a metal atom or an organic cation; R⁵ is a straight chain or branched chain alkyl group represented by the formula CᵣH₂ᵣ₊₁, wherein r is an integer of 4 to 48; A is a straight-chain or branched chain alkylene group represented by the formula C_{q}H_{2q}, wherein q is an integer of 0 to 20; and p is ≥ 1.

Patent Document 4 discloses a gel composition having improved texture in use, the gel composition consisting of (a) an organosiloxane derivative, (b) a monohydric aliphatic alcohol having 10 to 30 carbon atoms, and (c) water. In one embodiment, the organosiloxane derivative is represented by the general formula Si(R¹)(R²)(R³)-(CH₂)₂-A-COOM, in which at least one of R¹ to R³ may be a functional group represented by -O-Si(R⁴)₃, wherein R⁴ is an alkyl group having 1 to 6 carbon atoms or a phenyl group, and the other(s) R¹ to R³ may be same or different and each may be a substituted or unsubstituted monovalent hydrocarbon group; M is a hydrogen atom, a metal atom, or an organic cation; and A is a linear or branched alkylene group represented by the formula C_{q}H_{2q}, wherein q is an integer of 0 to 20.

### Prior Art Documents

### Patent Documents

Patent Document 1: International Publication WO No. 2009/025146
Patent Document 2: European Patent Application Publication No. EP 2 181 697 A2
Patent Document 3: Japanese Patent Application Publication No. JP 2008-266285 A
Patent Document 4: European Patent Application Publication No. EP 2 174 985 A1

### Non Patent Document

Non Patent Document 1: "New Cosmetic Science, 2nd edition", 2001, edited by Takeo Mitsui, published by Nanzando, pp.497-504.

### Summary of Invention

### Problem to be solved by the Invention

The present invention has been completed in the view of the above conventional situations. An object of the present invention is to provide an oil-in-water type emulsified sunscreen cosmetic that, in spite of containing a large amount of an ultraviolet absorber and exhibiting a high defense effect against ultraviolet, has no stickiness on use, has good spreadability on the skin and a good affinity to the skin, and additionally, has excellent stability over time at low and high temperatures.

### Means for solving the Problem

The present inventor, as a result of intensive studies, has found that an large amount of an ultraviolet absorber can be added in an oil-in-water type emulsified sunscreen cosmetic without impairing the stability and the sense of use when mixing a specific organosiloxane derivative, a polyethylene glycol stearate, and a higher alcohol in a specific ratio, and thus have completed the present invention.

That is, the present invention relates to an oil-in-water type emulsified sunscreen cosmetic comprising:
(a) 0.1 to 5.0% by mass of 3-(10-carboxydecyl)-1,1,1,3,5,5,5-heptamethyltrisiloxane,
(b) 0.1 to 5.0% by mass of a polyethylene glycol stearate having 2 to 60 ethylene oxide units,
(c) 10.0 to 25.0% by mass of an ultraviolet absorber, and
(d) one or more higher alcohols selected from the group consisting of stearyl alcohol, isostearyl alcohol, oleyl alcohol, octyldodecanol, chimyl alcohol, cholesterol, sitosterol, cetanol, cetostearyl alcohol, selachyl alcohol, decyl tetradecanol, batyl alcohol, phytosterol, hexyldecanol, behenyl alcohol, lauryl alcohol, lanolin alcohol and hydrogenated lanolin alcohol.

### Effects of Invention

The oil-in-water type emulsified sunscreen cosmetic according to the present invention, in addition to having a high defense effect against ultraviolet, is stable even when stored under a low-temperature condition (about 0°C) and a high-temperature condition (about 50°C) for a long period, and additionally, has good spreadability on the skin and a good penetration ability to the skin, and has no stickiness after application on the skin, resulting in an excellent feeling of use.

### Modes for carrying out the invention

The oil-in-water type emulsified sunscreen cosmetic of the present invention is characteristic in that it comprises (a) an organosiloxane derivative, (b) a polyethylene glycol stearate, (c) an ultraviolet absorber, and (d) a higher alcohol in a specific ratio. Hereinafter, the present invention will be described in detail.

### <Component (a): an organosiloxane derivative>

A component (a), an organosiloxane derivative, used in the present invention is a compound having a silicone moiety and a carboxyl group on the skeleton.

Herein, the organosiloxane derivative is 3-(10-carboxydecyl)-1,1,1,3,5,5,5-heptamethyltrisiloxane (hereinafter, sometimes referred to as simply "carboxydecyl trisiloxane"), which is commercially available from Dow Corning Toray Co., Ltd. under the trade name "Dow Corning Toray OP-1800MF Carboxy Fluid".

The component (a), the organosiloxane derivative, is added in an amount of from 0.1 to 5.0% by mass, preferably from 0.1 to 2.0% by mass, relative to the total amount of the oil-in-water type emulsified sunscreen cosmetic. If the amount is less than 0.1% by mass, the stability over time becomes inferior, sometimes resulting in creaming due to significant increase or decrease of the viscosity particularly under high temperatures, and also stickiness after application on the skin tends to be pronounced. Meanwhile, if the component is added in an amount more than 5.0% by mass, the stability over time and the feeling of use are insufficient.

### <Component (b): a polyethylene glycol stearate>

A component (b), a polyethylene glycol stearate, used in the present invention is one having 2 to 60 ethylene oxide units (EOs), preferably one having 10 to 40 EOs. A particularly preferred (b) polyethylene glycol stearate is a polyethylene glycol monostearate (20 EOs).

Use of a polyethylene glycol stearate having less than 2 ethylene oxide units (EOs) may make the stability over time inferior. Meanwhile, use of a polyethylene glycol stearate having more than 60 ethylene oxide units may not provide refreshing sense on use and may cause sticky feeling.

The amount of the component (b), the polyethylene glycol stearate, to be added is from 0.1 to 5.0% by mass, preferably from 0.1 to 2.0% by mass, relative to the total amount of the oil-in-water type emulsified sunscreen cosmetic. If the amount is less than 0.1% by mass, the effects of the present invention when a large amount of an ultraviolet absorber is added, that is, excellent feeling of use and stability over time cannot be achieved, and particularly, an affinity to the skin tends to become inferior. Meanwhile, the effects cannot be enhanced even if the component is added more than 5.0% by mass, and it is not preferable because the feeling of use and the stability over time may be impaired.

### <Component (c): an ultraviolet absorber>

A component (c), an ultraviolet absorber, used in the present invention may be those conventionally used in cosmetics, and is not particularly limited. The examples thereof include methoxycinnamate derivatives, salicylate derivatives, benzoyl derivatives, camphor derivatives, para-aminobenzoate derivatives, triazine derivatives, benzophenone derivatives, diphenyl compounds, and polysilicones. Specifically, the examples thereof include 2-ethylhexyl para-methoxycinnamate, octocrylene, bis-ethylhexylphenol methoxyphenyl triazine, methylene bis-benzotriazolyl tetramethylbutylphenol, phenylbenzimidazole sulfonic acid, oxybenzone, 4-t-butyl-4'-methoxydibenzoylmethane, octyl triazone, dioctyl butamidotriazone, diethylamino hydroxybenzoyl hexyl benzoate, 2'-ethylhexyl 2-cyano-3,3-diphenylacrylate esters, Polysilicone-15, octyl salicylate, homomenthyl salicylate, and p-methylbenzylidenecamphor. One or more of these may be appropriately selected and used as required.

The amount of the component (c), the ultraviolet absorber, to be added in the present invention is from 10.0 to 25.0% by mass, preferably from 10.0 to 20.0% by mass, relative to the total amount of the oil-in-water type emulsified sunscreen cosmetic.
If the amount is less than 10.0% by mass, an excellent sunscreen effect cannot be achieved. If the amount of more than 25.0% by mass is added, the stability over time at low temperatures becomes inferior, and stickiness tends to be caused.

### <Component (d): a higher alcohol>

A component (d), a higher alcohol, used in the present invention is a saturated or unsaturated monovalent aliphatic alcohol having 10 to 30 carbon atoms, which may be straight or branched. A higher alcohol having a straight chain is preferred.

According to the invention, the higher alcohol is selected from the group consisting of stearyl alcohol, isostearyl alcohol, oleyl alcohol, octyldodecanol, chimyl alcohol, cholesterol, sitosterol, cetanol, cetostearyl alcohol, selachyl alcohol, decyl tetradecanol, batyl alcohol, phytosterol, hexyldecanol, behenyl alcohol, lauryl alcohol, lanolin alcohol and hydrogenated lanolin alcohol, and one or more of these are added.

The amount of the higher alcohol to be added is preferably from 0.1 to 10.0% by mass, more preferably from 0.3 to 5.0% by mass, relative to the total amount of the oil-in-water type emulsified sunscreen cosmetic. If the amount is less than 0.1% by mass, sufficient stability over time may not be achieved. If more than 10.0% by mass thereof is added, the stability over time tends to be impaired instead.

To the oil-in-water type emulsified sunscreen cosmetic according to the present invention, various components usually added in the field of cosmetics and quasi-pharmaceutical products, such as powder components, solid oils and fats, moisturizing agents, thickeners, metal ion sequestering agents, dyes, pH adjusting agents, skin nutrients, vitamins, preservatives, anti-oxidizing agents, anti-oxidizing aids, and perfumes can be appropriately added as required to the extent that the effects of the present invention are not impaired.

Of these, from a viewpoint of enhancing the beauty effect, medicinal agents generally used in sunscreen cosmetics are preferably added. Medicinal agents suitably added in the present invention may include whitening agents and anti-inflammatory agents. Examples of the whitening agent may include tranexamic acid, potassium 4-methoxysalicylate, and ascorbic acid glucoside, and examples of the anti-inflammatory agent include dipotassium glycyrrhizate.

The oil-in-water type emulsified sunscreen cosmetic according to the present invention can be produced in accordance with the methods conventionally used for producing emulsions. For example, oil phase components and water phase components are separately mixed to prepare an oil phase and a water phase, and then the water phase and the oil phase are mixed and emulsified in a homo-mixer or the like to thereby provide an oil-in-water type emulsified sunscreen cosmetic of the present invention.

### Examples

Hereinbelow, the present invention is described in more details referring to Examples, but the present invention is not intended to be limited to these Examples. The amount added represents in % by mass, unless otherwise indicated.

Oil-in-water type emulsified sunscreen cosmetics were prepared in accordance with the formulations shown in Tables 1 and 2 below and the production methods described below. The resulting oil-in-water type emulsified sunscreen cosmetics (samples) were examined for the stability over time and feeling of use (ease of spreading, quickness to develop an affinity, and stickiness) in accordance with the evaluation method below. The results are shown together in Tables 1 and 2.

### [Stability over time test]

### (Stability at low temperatures)

The samples were placed in a thermostatic chamber at 0°C and left for four weeks. Then, the state at 0°C was examined with a Brookfield type viscometer, and evaluated in accordance with the evaluation criteria below.

### (Evaluation criteria)

○: No increases or decreases in the viscosity were observed at all.
Δ: Slight increases or decreases in the viscosity were observed.
×: Significant increases or decreases in the viscosity were observed.

### (Stability at high temperatures)

The samples were placed in a thermostatic chamber at 50°C and left for four weeks. Then, the state at 50°C was examined with a Brookfield type viscometer and visual inspection, and evaluated in accordance with the evaluation criteria below.

### (Evaluation criteria)

○: No increases or decreases in the viscosity were observed at all.
Δ: Slight increases or decreases in the viscosity were observed.
×: Significant increases or decreases in the viscosity and creaming were observed.

### [Test for feeling of use]

Ten specialized panelists applied each sample on the face, and evaluated for ease of spreading during application (a penetration feeling), quickness to develop an affinity, and stickiness after application in accordance with the evaluation criteria below.

### (Spreadability: Evaluation criteria)

⊚: Nine to ten panelists answered that the spreadability was good.
○: Six to eight panelists answered that the spreadability was good.
Δ: Three to five panelists answered that the spreadability was good.
×: Zero to two panelists answered that the spreadability was good.

### (Affinity: Evaluation criteria)

⊚: Nine to ten panelists answered that the affinity was good.
○: Six to eight panelists answered that the affinity was good.
Δ: Three to five panelists answered that the affinity was good.
×: Zero to two panelists answered that the affinity was good.

### (Stickiness: Evaluation criteria)

⊚: Nine to ten panelists answered that they found no stickiness.
○: Six to eight panelists answered that they found no stickiness.
Δ: Three to five panelists answered that they found no stickiness.
×: Zero to two panelists answered that they found no stickiness.

**[Table 1]**

| | Component (% by mass) | Example 1 | Example 2 | Example 3 | Example 4 | Example 5 | Example 6 | Example 7 |
|---|---|---|---|---|---|---|---|---|
| (1) | Ion-exchanged | Balance | Balance | Balance | Balance | Balance | Balance | Balance |
| (2) | Ethanol | 3 | 3 | 3 | 3 | 3 | 3 | 3 |
| (3) | Glycerin | 2 | 2 | 2 | 2 | 2 | 2 | 2 |
| (4) | Dipropylene glycol | 5 | 5 | 5 | 5 | 5 | 5 | 5 |
| (5) | Triethanolamine | 1.5 | 1.5 | 1.5 | 1.5 | 1.5 | 1.5 | 1.5 |
| (6) | Succinoglycan | 0.2 | 0.2 | 0.2 | 0.2 | 0.2 | 0.2 | 0.2 |
| (7) | Carboxydecyl trisiloxane | 0.1 | 1 | 3 | 5 | 1 | 1 | 1 |
| (8) | Glyceryl monostearate | 0.2 | 0.2 | 0.2 | 0.2 | 0.2 | 0.2 | 0.2 |
| (9) | Sorbitan monostearate | 0.1 | 0.1 | 0.1 | 0.1 | 0.1 | 0.1 | 0.1 |
| (10) | Polyethylene glycol monostearate (20E.O.) | 1 | 1 | 1 | 1 | 0.2 | 3 | 5 |
| (11) | Behenyl alcohol | 2 | 2 | 2 | 2 | 2 | 2 | 2 |
| (12) | Batyl alcohol | 1 | 1 | 1 | 1 | 1 | 1 | 1 |
| (13) | Caprylyl methicone | 3 | 3 | 3 | 3 | 3 | 3 | 3 |
| (14) | Dimethyl polysiloxane (6cs) | 7 | 7 | 7 | 7 | 7 | 7 | 7 |
| (15) | Decamethyl tetrasiloxane | 7 | 7 | 7 | 7 | 7 | 7 | 7 |
| (16) | Phenyl trimethicone | 2 | 2 | 2 | 2 | 2 | 2 | 2 |
| (17) | Polyoxybutylene polyoxypropylene glycol | 2 | 2 | 2 | 2 | 2 | 2 | 2 |
| (18) | Bis-ethylhexyloxyphenol methoxyphenyl triazine | 3 | 3 | 3 | 3 | 3 | 3 | 3 |
| (19) | Octocrylene | 3 | 3 | 3 | 3 | 3 | 3 | 3 |
| (20) | 2-ethylhexyl para-methoxycinnamate | 7 | 7 | 7 | 7 | 7 | 7 | 7 |
| (21) | Phenyl benzimidazole sulfonic acid | 2 | 2 | 2 | 2 | 2 | 2 | 2 |
| (22) | Methylene bis-benzotriazolyl tetramethylbutylphenol | 2 | 2 | 2 | 2 | 2 | 2 | 2 |
| (23) | Phenoxyethanol | Appropriate amount | Appropriate amount | Appropriate amount | Appropriate amount | Appropriate amount | Appropriate amount | Appropriate amount |
| (24) | Disodium edetate | Appropriate amount | Appropriate amount | Appropriate amount | Appropriate amount | Appropriate amount | Appropriate amount | Appropriate amount |
| Total | | 100 | 100 | 100 | 100 | 100 | 100 | 100 |
| Evaluation | | | | | | | | |
| Stability over time | Stability over time (Viscosity at 0°C) | ○ | ○ | ○ | ○ | ○ | ○ | ○ |
| | Stability over time (Viscosity at 50°C, creaming) | ○ | ○ | ○ | ○ | ○ | ○ | ○ |
| Feeling of use | Spreadability | ○ | ⊚ | ○ | ○ | ○ | ⊚ | ○ |
| | Affinity | ○ | ⊚ | ○ | ○ | ○ | ⊚ | ○ |
| | Stickiness | ○ | ⊚ | ⊚ | ○ | ○ | ○ | ○ |

### Production method:

The components (1) to (6), (23) and (24) in Table 1 were mixed and dissolved at normal temperature to thereby obtain a water phase. Meanwhile, the components (7) to (22) were dissolved under heating at 70°C to thereby obtain an oil phase. Subsequently, the water phase was heated to 70°C, and the oil phase was gradually added thereto. The resultant was stirred and mixed by using a homo-mixer, and the mixture was allowed to return to room temperature to thereby obtain the cosmetics of Examples 1 to 7.

**[Table 2]**

| | Component (% by mass) | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|
| (1) | Ion-exchanged water | Balance | Balance | Balance | Balance | Balance | Balance | Balance | Balance | Balance | Balance |
| (2) | Ethanol | 3 | 3 | 3 | 3 | 3 | 3 | 3 | 3 | 3 | 3 |
| (3) | Glycerin | 2 | 2 | 2 | 2 | 2 | 2 | 2 | 2 | 2 | 2 |
| (4) | Dipropylene glycol | 5 | 5 | 5 | 5 | 5 | 5 | 5 | 5 | 5 | 5 |
| (5) | Triethanolamine | 1.5 | 1.5 | 1.5 | 1.5 | 1.5 | 1.5 | 1.5 | 1.5 | 1.5 | 1.5 |
| (6) | Succinoglycan | 0.2 | 0.2 | 0.2 | 0.2 | 0.2 | 0.2 | 0.2 | 0.2 | 0.2 | 0.2 |
| (7) | Carboxydecyl trisiloxane | 0.05 | 6 | 1 | 1 | - | - | 1 | 1 | 1 | 1 |
| (8) | Glyceryl monostearate | 0.2 | 0.2 | 0.2 | 0.2 | 1 | 1 | 0.2 | 0.2 | 0.2 | 0.2 |
| (9) | Sorbitan monostearate | 0.1 | 0.1 | 0.1 | 0.1 | - | - | 0.1 | 0.1 | 0.1 | 0.1 |
| (10) | Polyoxyethylene glyceryl monostearate (5E.O.) | - | - | - | - | 1 | - | - | - | - | - |
| (11) | Polyoxyethylene glyceryl isostearate (60E.O.) | - | - | - | - | - | 1 | - | - | - | - |
| (12) | Propylene glycol stearate | - | - | - | - | - | 1 | - | - | - | - |
| (13) | Polyethylene glycol monostearate (20E.O.) | 1 | 1 | 0.05 | 6 | 1 | - | - | - | 1 | 1 |
| (14) | Polyoxyethylene behenyl ether (20E.O.) | - | - | - | - | - | - | 1 | - | - | - |
| (15) | Polyoxyethylene sorbitan monostearate (20E.O.) | - | - | - | - | - | - | - | 1 | - | - |
| (16) | Behenic acid | - | - | - | - | 0.3 | 1 | - | - | - | - |
| (17) | Isostearic acid | - | - | - | - | - | - | - | - | - | - |
| (18) | Behenyl alcohol | 2 | 2 | 2 | 2 | - | 1 | 2 | 2 | 2 | 2 |
| (19) | Batyl alcohol | 1 | 1 | 1 | 1 | - | 0.5 | 1 | - | 1 | 1 |
| (20) | Caprylyl methicone | 3 | 3 | 3 | 3 | 3 | 3 | 3 | 3 | 3 | 3 |
| (21) | Dimethyl polysiloxane (6cs) | 7 | 7 | 7 | 7 | 7 | 7 | 7 | 7 | 7 | 7 |
| (22) | Decamethyl tetrasiloxane | 7 | 7 | 7 | 7 | 7 | 7 | 7 | 7 | 7 | 7 |
| (23) | Phenyl trimethicone | 2 | 2 | 2 | 2 | 2 | 2 | 2 | 2 | 2 | 2 |
| (24) | Polyoxybutylene polyoxypropylene glycol | 2 | 2 | 2 | 2 | 2 | 2 | 2 | 2 | 2 | 2 |
| (25) | Bis-ethylhexyloxyphenol methoxyphenyl triazine | 3 | 3 | 3 | 3 | 3 | 3 | 3 | 3 | 1 | 4 |
| (26) | Octocrylene | 3 | 3 | 3 | 3 | 3 | 3 | 3 | 3 | 1 | 4 |
| (27) | 2-ethylhexyl para-methoxycinnamate | 7 | 7 | 7 | 7 | 7 | 7 | 7 | 7 | 4 | 12 |
| (28) | Phenyl benzimidazole sulfonic acid | 2 | 2 | 1 | 2 | 2 | 2 | 2 | 2 | 1 | 4 |
| (29) | Methylene bis-benzotriazolyl tetramethylbutylphenol | 2 | 2 | 2 | 2 | 2 | 2 | 2 | 2 | 1 | 4 |
| (30) | Phenoxyethanol | Appropriate amount | Appropriate amount | Appropriate amount | Appropriate amount | Appropriate amount | Appropriate amount | Appropriate amount | Appropriate amount | Appropriate amount | Appropriate amount |
| (31) | Disodium edetate | Appropriate amount | Appropriate amount | Appropriate amount | Appropriate amount | Appropriate amount | Appropriate amount | Appropriate amount | Appropriate amount | Appropriate amount | Appropriate amount |
| Total | | 100 | 100 | 100 | 100 | 100 | 100 | 100 | 100 | 100 | 100 |
| Evaluation | | | | | | | | | | | |
| Stability over time | Stability over time(Viscosity at 0°C) | Δ | Δ | Δ | Δ | Δ | Δ | ○ | ○ | ○ | × |
| | Stability over time (Viscosity at 50°C, creaming) | × | Δ | × | Δ | Δ | Δ | ○ | ○ | ○ | Δ |
| Feeling of use | Spreadability | Δ | Δ | Δ | Δ | Δ | Δ | Δ | Δ | ⊚ | ○ |
| | Affinity | Δ | Δ | × | Δ | Δ | Δ | Δ | Δ | ⊚ | ○ |
| | Stickiness | × | Δ | Δ | Δ | Δ | × | Δ | Δ | ⊚ | Δ |

The components (1) to (6), (30) and (31) in Table 2 were mixed and dissolved at normal temperature to thereby obtain a water phase. Meanwhile, the components (7) to (29) were dissolved under heating at 70°C to thereby obtain an oil phase. Subsequently, the water phase was heated to 70°C, and the oil phase was gradually added thereto. The resultant was stirred and mixed by using a homo-mixer, and the mixture was allowed to return to room temperature to thereby provide the cosmetics of Comparative Examples 1 to 10.

It can be seen from the result in Table 1 that the oil-in-water type emulsified sunscreen cosmetics of Examples 1 to 7 according to the present invention had excellent stability over time at both low and high temperatures and exhibited no significant increases and decreases in the viscosity, although a large amount of an ultraviolet absorber was added. Additionally, it was shown that these cosmetics were highly evaluated in ease of spreading on the skin, quickness to develop an affinity to the skin, and stickiness, that is, these cosmetics had an excellent feeling of use.

In contrast, it was shown from the result of Table 2 that in the case where the amount of the added organosiloxane derivative (component (a)) was too little or too much (Comparative Examples 1 and 2), in the case where the amount of the added polyethylene glycol monostearate (20 EOs) (component (b)) was too little or too much (Comparative Examples 3 and 4), in the case where one or both of the component (a) and component (b) were not contained (Comparative Examples 5 to 8), and in the case where the amount of the added ultraviolet absorber (component (c)) was too much (Comparative Example 10), either the stability over time or the feeling of use was inferior or both of them were inferior. It should be noted that numerical values such as SPFs are not shown in the Tables above, but that in Comparative Example 9, although excellent results were shown for both of the stability over time and the feeling of use, the sunscreen effect was not sufficient because the amount of (c) the ultraviolet absorber added was small.

### (Formulation examples)

Formulation examples of the oil-in-water type emulsified sunscreen cosmetic of the present invention are listed below. It goes without saying that the present invention is not intended to be limited to the formulation examples, but is identified by the claims. It should be noted that all the amounts added are represented in % by mass based on the total amount of the product.

**Formulation Example 1. Sunscreen cosmetic**

| (Component) | Amount added (%) |
|---|---|
| (1) Polyoxyethylene hydrogenated castoroil (60 EOs) | 1 |
| (2) Dimethicone polyol | 0.5 |
| (3) Decamethyl cyclopentasiloxane | 15 |
| (4) Carboxydecyl trisiloxane | 0.5 |
| (5) Polyethylene glycol monostearate (20 EOs) | 0.5 |
| (6) Behenyl alcohol | 0.3 |
| (7) Phenyl trimethicone | 1 |
| (8) Fine titanium dioxide | 5 |
| (9) Fine zinc oxide | 2 |
| (10) Crosslinked PMMA powder | 2 |
| (11) 2-ethylhexyl para-methoxycinnamate | 10 |
| (12) Citric acid | 0.01 |
| (13) Sodium citrate | 0.09 |
| (14) Silica | 1 |
| (15) Paraben | Appropriate amount |
| (16) Phenoxyethanol | Appropriate amount |
| (17) Sodium hydroxide | 0.05 |
| (18) Ethanol | 5 |
| (19) Glycerin | 1 |
| (20) Succinoglucan | 0.2 |
| (21) Cellulose gum | 1 |
| (22) Ion-exchanged water | Balance |

### [Production method]

The components (12) to (22) were mixed and dissolved at normal temperature to thereby obtain a water phase. Meanwhile, the components (1) to (11) were dissolved under heating at 70°C to thereby obtain an oil phase. Subsequently, the water phase was heated to 70°C, and the oil phase was gradually added thereto. After the resultant was stirred and mixed by using a homo-mixer, the mixture was cooled to room temperature to thereby obtain the sunscreen cosmetic of Formulation Example 1. When the obtained sunscreen cosmetic was subjected to the same evaluation as in Examples, the cosmetic showed excellent stability over time both at low and high temperatures (stability evaluation: ○) and excellent feeling of use (evaluation of feeling of use: ○ or ⊚) for all of the spreadability on the skin, the affinity to the skin, and no stickiness after application.)

**Formulation Example 2 Sunscreen cosmetic**

| (Component) | Amount added (%) |
|---|---|
| (1) Ion-exchanged water | Balance |
| (2) Dipropylene glycol | 7 |
| (3) Glycerin | 2 |
| (4) Carboxy vinyl polymer | 0.1 |
| (5) Potassium hydroxide | Appropriate amount |
| (6) Carboxydecyl trisiloxane | 1 |
| (7) Glyceryl monostearate | 2 |
| (8) Polyethylene glycol monostearate (20 EOs) | 0.5 |
| (9) Batyl alcohol | 0.2 |
| (10) Behenyl alcohol | 1 |
| (11) Decamethyl cyclopentasiloxane | 3 |
| (12) Dimethyl polysiloxane | 1 |
| (13) Phenyl trimethicone | 1 |
| (14) Octocrylene | 5 |
| (15) 2-ethylhexyl para-methoxycinnamate | 8 |
| (16) Disodium edetate | Appropriate amount |
| (17) Phenoxyethanol | Appropriate amount |

### [Production method]

The components (1) to (5), (16) and (17) were mixed and dissolved at normal temperature to thereby obtain a water phase. Meanwhile, the components (6) to (15) were dissolved under heating at 70°C to thereby obtain an oil phase. Subsequently, the water phase was heated to 70°C, and the oil phase was gradually added thereto. After the resultant was stirred and mixed by using a homo-mixer, the mixture was cooled to room temperature to thereby obtain the sunscreen cosmetic of Formulation Example 2. When the obtained sunscreen cosmetic was subjected to the same evaluation as in Examples, the cosmetic showed excellent stability over time both at low and high temperatures (stability evaluation: ○) and excellent feeling of use (evaluation of feeling of use: ○ or ⊚ for all of the spreadability on the skin, the affinity to the skin, and no stickiness after application.)

**Formulation Example 3. Cosmetic primer**

| (Component) | Amount added (%) |
|---|---|
| (1) Ion-exchanged water | Balance |
| (2) Ethanol | 3 |
| (3) Glycerin | 5 |
| (4) Triethanolamine | 1.5 |
| (5) Rheozan | 0.2 |
| (6) Carboxydecyl trisiloxane | 1 |
| (7) Glyceryl monostearate | 0.2 |
| (8) Sorbitan monostearate | 1.1 |
| (9) Polyethylene glycol monostearate (20 EOs) | 1 |
| (10) Behenyl alcohol | 2 |
| (11) Batyl alcohol | 1 |
| (12) Decamethyl tetrasiloxane | 7 |
| (13) Phenyl trimethicone | 5 |
| (14) Octocrylene | 5 |
| (15) 2-ethylhexyl para-methoxycinnamate | 8 |
| (16) Iron oxide | Appropriate amount |
| (17) Phenoxyethanol | Appropriate amount |
| (18) Disodium edetate | Appropriate amount |
| (19) Sodium metaphosphate | Appropriate amount |

### [Production method]

The components (1) to (5), (17) to (19) were mixed and dissolved at normal temperature to thereby obtain a water phase. Meanwhile, the components (6) to (15) were dissolved under heating at 70°C to thereby obtain an oil phase. Subsequently, the water phase was heated to 70°C, the oil phase was gradually added thereto, and a component (16) was further added. After the resultant was stirred and mixed by using a homo-mixer, the mixture was cooled to room temperature to thereby obtain the cosmetic primer of Formulation Example 3. When the obtained cosmetic primer was subjected to the same evaluation as in Examples, the cosmetic primer showed excellent stability over time both at low and high temperatures (stability evaluation: ○) and excellent feeling of use (evaluation of feeling of use: ○ or ⊚ for all of the spreadability on the skin, the affinity to the skin, and no stickiness after application.)

**Formulation Example 4. Whitening sunscreen cosmetic**

| (Component) | Amount added (%) |
|---|---|
| (1) Polyoxyethylene hydrogenated castor oil (60 EOs) | 1 |
| (2) Dimethicone polyol | 0.5 |
| (3) Decamethyl cyclopentasiloxane | 15 |
| (4) Carboxydecyl trisiloxane | 0.5 |
| (5) Polyethylene glycol monostearate (20 EOs) | 0.5 |
| (6) Behenyl alcohol | 0.3 |
| (7) Phenyl trimethicone | 1 |
| (8) Fine titanium dioxide | 5 |
| (9) Fine zinc oxide | 2 |
| (10) Crosslinked PMMA powder | 2 |
| (11) 2-Ethylhexyl para-methoxycinnamate | 10 |
| (12) Citric acid | Appropriate amount |
| (13) Sodium citrate | Appropriate amount |
| (14) Silica | 1 |
| (15) Paraben | Appropriate amount |
| (16) Phenoxyethanol | Appropriate amount |
| (17) Sodium hydroxide | 0.05 |
| (18) Ethanol | 5 |
| (19) Glycerin | 1 |
| (20) Succinoglucan | 0.2 |
| (21) Cellulose gum | 1 |
| (22) Tranexamic acid | 2 |
| (23) Ion-exchanged water | Balance |

### [Production method]

The components (11) to (23) were mixed and dissolved at normal temperature to thereby obtain a water phase. Meanwhile, the components (1) to (10) were mixed to thereby obtain an oil phase. Subsequently, the water phase was gradually added to the oil phase. The resultant was stirred and mixed by using a homo-mixer to thereby obtain the whitening sunscreen cosmetic of Formulation Example 4. When the obtained cosmetic was subjected to the same evaluation as in Examples, the cosmetic showed excellent stability over time both at low and high temperatures (stability evaluation: ○) and excellent feeling of use (evaluation of feeling of use: ○ or ⊚ for all of the spreadability on the skin, the affinity to the skin, and no stickiness after application.)

**Formulation Example 5. Whitening sunscreen cosmetic**

| (Component) | Amount added (%) |
|---|---|
| (1) Ion-exchanged water | Balance |
| (2) Dipropylene glycol | 7 |
| (3) Glycerin | 2 |
| (4) Carboxy vinyl polymer | 0.1 |
| (5) Potassium hydroxide | Appropriate amount |
| (6) Carboxydecyl trisiloxane | 1 |
| (7) Glycerin monostearate | 2 |
| (8) Polyethylene glycol monostearate (20 EOs) | 0.5 |
| (9) Batyl alcohol | 0.2 |
| (10) Behenyl alcohol | 1 |
| (11) Decamethyl cyclopentasiloxane | 3 |
| (12) Dimethyl polysiloxane | 1 |
| (13) Phenyl trimethicone | 1 |
| (14) Octocrylene | 5 |
| (15) 2-ethylhexyl para-methoxycinnamate | 8 |
| (16) Disodium edetate | Appropriate amount |
| (17) Phenoxyethanol | Appropriate amount |
| (18) Potassium 4-methoxysalicylate | 1 |

### [Production method]

After the component (18) was dissolved in the component (1), the components (2) to (5), (16) and (17) were mixed thereto to thereby obtain a water phase. Meanwhile, the components (6) to (15) were dissolved under heating at 70°C to thereby obtain an oil phase. Subsequently, the water phase was heated to 70°C, and the oil phase was gradually added thereto. After the resultant was stirred and mixed by using a homo-mixer, the mixture was cooled to room temperature to thereby obtain the whitening sunscreen cosmetic of Formulation Example 5. When the obtained cosmetic was subjected to the same evaluation as in Examples, the cosmetic showed excellent stability over time both at low and high temperatures (stability evaluation: ○) and excellent feeling of use (evaluation of feeling of use: ○ or ⊚ for all of the spreadability on the skin, the affinity to the skin, and no stickiness after application.)

**Formulation Example 6. Whitening cosmetic primer**

| (Component) | Amount added (%) |
|---|---|
| (1) Ion-exchanged water | Balance |
| (2) Ethanol | 3 |
| (3) Glycerin | 5 |
| (4) Triethanolamine | 1.5 |
| (5) Rheozan | 0.2 |
| (6) Carboxydecyl trisiloxane | 1 |
| (7) Glycerin monostearate | 0.2 |
| (8) Sorbitan monostearate | 1.1 |
| (9) Polyethylene glycol monostearate (20 EOs) | 1 |
| (10) Behenyl alcohol | 2 |
| (11) Batyl alcohol | 1 |
| (12) Decamethyl tetrasiloxane | 7 |
| (13) Phenyl trimethicone | 5 |
| (14) Octocrylene | 5 |
| (15) 2-Ethylhexyl para-methoxycinnamate | 8 |
| (16) Iron oxide | Appropriate amount |
| (17) Phenoxyethanol | Appropriate amount |
| (18) Disodium edetate | Appropriate amount |
| (19) Sodium metaphosphate | Appropriate amount |
| (20) Dipotassium glycyrrhizate | 0.05 |
| (21) Ascorbic acid glucoside | 2 |
| (22) Potassium hydroxide | Appropriate amount |
| (23) Citric acid | Appropriate amount |
| (24) Sodium citrate | Appropriate amount |

### [Production method]

After the components (20) to (24) were dissolved in the components (1), the components (2) to (5) and (17) to (19) were mixed thereto to thereby obtain a water phase. Meanwhile, the components (6) to (15) were dissolved under heating at 70°C to thereby obtain an oil phase. Subsequently, the water phase was heated to 70°C, the oil phase was gradually added thereto, and the component (16) was further added. After the resultant was stirred and mixed by using a homo-mixer, the mixture was cooled to room temperature to thereby obtain the whitening cosmetic primer of Formulation Example 6. When the obtained cosmetic primer was subjected to the same evaluation as in Examples, the cosmetic primer showed excellent stability over time both at low and high temperatures (stability evaluation: ○) and excellent feeling of use (evaluation of feeling of use: ○ or ⊚ for all of the spreadability on the skin, the affinity to the skin, and no stickiness after application.)

## Claims

1. An oil-in-water type emulsified sunscreen cosmetic comprising:
(a) 0.1 to 5.0% by mass of 3-(10-carboxydecyl)-1,1,1,3,5,5,5-heptamethyltrisiloxane,
(b) 0.1 to 5.0% by mass of a polyethylene glycol stearate having 2 to 60 ethylene oxide units,
(c) 10.0 to 25.0% by mass of an ultraviolet absorber, and
(d) one or more higher alcohols selected from the group consisting of stearyl alcohol, isostearyl alcohol, oleyl alcohol, octyldodecanol, chimyl alcohol, cholesterol, sitosterol, cetanol, cetostearyl alcohol, selachyl alcohol, decyl tetradecanol, batyl alcohol, phytosterol, hexyldecanol, behenyl alcohol, lauryl alcohol, lanolin alcohol and hydrogenated lanolin alcohol.

## Patentansprüche

1. Sonnenschutzkosmetikum in Form einer Öl-in-Wasser-Emulsion, umfassend:
(a) 0.1 bis 5.0 Masse% an 3-(10-Carboxydecyl)-1,1,1,3,5,5,5-heptamethyltrisiloxan,
(b) 0.1 bis 5.0 Masse% an einem Polyethylenglykolstearat mit 2 bis 60 Ethylenoxideinheiten,
(c) 10.0 bis 25.0 Masse% an einem Ultraviolettabsorber, und
(d) einen oder mehrere höhere Alkohole ausgewählt aus der Gruppe bestehend aus Stearylalkohol, Isostearylalkohol, Oleylalkohol, Octyldodecanol, Chimylalkohol, Cholesterol, Sitosterol, Cetanol, Cetostearylalkohol, Selachylalkohol, Decyltetradecanol, Batylalkohol, Phytosterol, Hexyldecanol, Behenylalkohol, Laurylalkohol, Lanolinalkohol und hydriertem Lanolinalkohol.

## Revendications

1. Produit cosmétique écran solaire émulsifié de type huile-dans-l'eau comprenant :
(a) 0,1 à 5,0 % en masse de 3-(10-carboxydécyl)-1,1,1,3,5,5,5-heptaméthyltrisiloxane,
(b) 0,1 à 5,0 % en masse de stéarate de polyéthylène glycol ayant 2 à 60 motifs d'oxyde d'éthylène,
(c) 10,0 à 25,0 % en masse d'un absorbeur d'ultraviolets, et
(d) un ou plusieurs alcools supérieurs sélectionnés dans le groupe constitué de l'alcool stéarylique, de l'alcool isostéarylique, de l'alcool oléylique, de l'octyldodécanol, de l'alcool chimylique, du cholestérol, du sitostérol, du cétanol, de l'alcool cétostéarylique, de l'alcool sélachylique, du décyl tétradécanol, de l'alcool batylique, du phytostérol, de l'hexyldécanol, de l'alcool béhénylique, de l'alcool laurylique, de l'alcool lanolinique et de l'alcool lanolinique hydrogéné.
